(19) **Europäisches Patentamt / European Patent Office / Office européen des brevets**

(11) **EP 1 865 008 A1**

(12) **EUROPEAN PATENT APPLICATION**

(43) Date of publication:
**12.12.2007 Bulletin 2007/50**

(21) Application number: **07010166.2**

(22) Date of filing: **22.05.2007**

(51) Int Cl.:
**C08F 251/00** (2006.01)   **C08F 251/02** (2006.01)
**C08F 265/04** (2006.01)   **C08F 265/06** (2006.01)
**C08F 283/00** (2006.01)   **C08L 51/02** (2006.01)
**C09D 151/02** (2006.01)   **C09J 151/02** (2006.01)
**C08L 1/00** (2006.01)   **C08L 3/00** (2006.01)
**C08L 5/00** (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **05.06.2006 KR 20060050396**

(71) Applicant: **Duksung Co., Ltd.**
**Yeongtong-gu**
**Suwon-si, Gyeonggi-do**
**443-390 (KR)**

(72) Inventors:
• **Lee, Soo-Chang**
  **Gyeonggi-do 443-400 (KR)**
• **Kim, Geel-Ja**
  **Gyeongsangbuk-do 790-150 (KR)**

(74) Representative: **Scheele, Friedrich et al**
**Stolmár Scheele & Partner**
**Blumenstrasse 17**
**80331 München (DE)**

(54) **Method for preparing high absorbent hydrocolloid**

(57) The present invention relates to a method for preparing a hydrocolloid which may be applied to skin, particularly, wounds. The present invention comprises a first step of preparing a pre-polymer having a viscosity of 200 cps - 10,000 cps by mixing an acryl monomer and an ultraviolet initiator and irradiating on the mixture with ultraviolet rays; a second step of preparing a complex by mixing at least an ultraviolet initiator and a high water absorbing substance with the resulting pre-polymer; and a third step of polymerizing the complex with irradiating ultraviolet rays. The thus prepared hydrocolloid of the present invention has an excellent absorbency as well as self-adhesiveness, is not remained as any residue upon removing it from the skin (wound) and has less skin irritation, without employing any tackifier.

Fig. 1

EP 1 865 008 A1

## Description

### FIELD OF THE INVENTION

The present invention relates to a method for preparing a hydrocolloid which may be usefully applied to the skin, particularly wounds. More specifically, the present invention relates to a method for preparing a high absorbent hydrocolloid which has self-adhesiveness and an excellent absorbency, is not allowed to leave any residue upon removing it from the skin (wound), and has less skin stimuli, by mixing an acrylic pre-polymer with an ultraviolet initiator and a high absorbent substance and polymerizing the mixture through ultraviolet irradiation.

### BACKGROUND OF THE INVENTION

Skin protects human body from external stimuli and has functions such as moisture maintenance, temperature regulation, and prohibition from bacteria invasion. If skin loses such functions due to wounds such as various traumas, bums and bedsores, then patients suffer pain until they are completely recovered. When the patients are widely damaged in the skin, they are life-threatened. To promote treatment of wounds and minimize a variety of secondary side effects, it is necessary to treat the wounds using appropriate wound dressings.

Generally, wound dressings were not so self-adhesive that they might be mainly adhered and fixed to a wound (trauma) by a separate adhesive tape. However, it is troublesome to use the dressings. Thus, it is recently noted for a wound dressing with self-adhesiveness. A representative example is a hydrocolloid. Hydrocolloids have self-adhesiveness, and thus may be usefully used as wound dressings. A number of prior art patent publications are disclosed.

Hydrocolloids were initially prepared using compositions in which a low molecular weight polyisobutlylene was mixed with an absorbent, a low molecular tackifier and a plasticizer. Such a method is disclosed in US Patent No. 3,339,546. The thus prepared hydrocolloid was kept in a wet environment upon being applied to the skin, and was conveniently used due to self-adhesiveness. However, there were problems that the hydrocolloid was so opaque that the wounds could not be easily observed and that the low molecular weight polyisobuthylene was remained on the wounds in the form of gel.

In US Patent No. 4,231,369, a hydrocolloid prepared by mixing styrene-isoprene-styrene copolymer with a low molecular weight tackifier and a plasticizer, and an additive such as an antioxidant was disclosed. In addition, a hydrocolloid prepared by mixing a blend of polyisobutylene and butyl rubber with a styrene copolymer, mineral oil, a tackifier and an absorbent was disclosed in US Patent No. 4,551,490. In US Patent No. 6,583,220, a hydrocolloid prepared by mixing styrene-isoprene-styrene copolymer with a liquid styrene-isoprene rubber, a water soluble polymer, is disclosed.

The styrene-isoprene-styrene copolymer maintains a physically cross-linked structure on being applied to a wound region, and is not remained on the wound. However, the hydrocolloids disclosed in the prior art patents above are also so opaque that the wound cannot be easily observed. In addition, there are problems that absorbency of the hydrocolloids disclosed in the prior art patents above is lowered and skin stimuli are caused by the low molecular weight hydrocarbon, and the like.

In US Patent Nos. 4,477,325 and 4,738,257, a chemically cross-linked structure was prepared by adding a low molecular polyisobutylene and an absorbent to ethylene-vinyl acetate (EVA) copolymer and cross-linking the EVA with gamma ray irradiation. There was a problem that the absorbing performance could be varied with causing the deviations of cross-linking density depending on deviations of gamma ray radiation doses

Meanwhile, acrylate polymers have a good self-adhesiveness and thus are widely used as adhesives in many fields. When the acrylate polymer has an appropriate glass transition temperature (Tg), it has an excellent adhesiveness. The acrylate polymer has an advantage to be clear. In polymerization of acrylate polymers, acrylate based monomers such as n-butyl acrylate, 2-ethylhexyl acrylate, and isooctyl acrylate are mainly used. In case of polymerizing the acrylate monomer only, the resulting polymer is less absorbent. Generally, absorbency is obtained by adding a hydrophilic monomer having an acid group and polymerizing the mixture. However, in case of adding the hydrophilic monomer having an acid group, a problem of lowering adhesiveness was accompanied.

In US Patent No. 6,903,151, an adhesive prepared by adding a hydrophilic monomer having an acid group such as carboxylic acid, sulfonic acid, and phosphonic acid, and a polymer of alkylene oxide, such as ethylene oxide and propylene oxide, for adhesiveness to an acrylate based monomer such as n-butyl acrylate, 2-ethylhexyl acrylate, and isooctyl acrylate, is disclosed as an appropriate adhesive for wet skin. The adhesive has a low absorbency, and thus it is difficult to apply to the wound causing many exudates. In addition, it should be coated on objects for coating process, such as films. At this time, its physical properties such as a coating property by a large quantity of solvent and adhesiveness by an additive, for example, alkylene oxide polymer, are lowered, and the processes for preparing it are complicated. Furthermore, it is pointed out about problems that an additive, such as alkylene oxide polymer, may be leaked to leave residues on the skin (wound) and to cause skin stimuli.

Therefore, to apply an adhesive to the skin, particularly wounds, it should have a self adhesiveness to be conveniently used, have an excellent absorbency of exudates, do not leave residues upon being removed from the skin (wound), and have less skin stimuli as well.

## SUMMARY OF THE INVENTION

To solve the prior art problems as described above and the technical subjects, the object of the present invention is to provide an improved method for preparing a high absorbent hydrocolloid having a self-adhesiveness and an excellent absorbency, leaving no residue upon being removed from skin (wound), and having less skin stimuli without any low molecular weight tackifier, which comprises mixing an acrylic polymer with an ultraviolet initiator and a high absorbent substance, and polymerizing the mixture through ultraviolet irradiation.

To achieve this object, the present invention includes a method for a hydrocolloid which comprises
a first step of preparing a pre-polymer having a viscosity of 200 cps ~ 10,000 cps by mixing an acryl monomer and an ultraviolet initiator and irradiating on the mixture with ultraviolet rays;
a second step of preparing a complex by mixing the resulting pre-polymer with at least an ultraviolet initiator and a high absorbent substance; and
a third step of polymerizing the resulting complex by coating on an object for coating process with the complex and irradiating the coated complex with ultraviolet rays.

Preferably, the present invention further comprises a step of inhibiting the polymerization using oxygen, when the temperature after irradiating ultraviolet rays raises 5 °C ~ 20°C more than that before the irradiation upon preparing the pre-polymer in the first step. At this time, the efficiency of polymerization in the third step may be dropped by oxygen used in said step. Thus, the present invention further, preferably, comprises a step of removing oxygen in the complex using an inert gas.

## BRIEF DESCRIPTION OF DRAWINGS

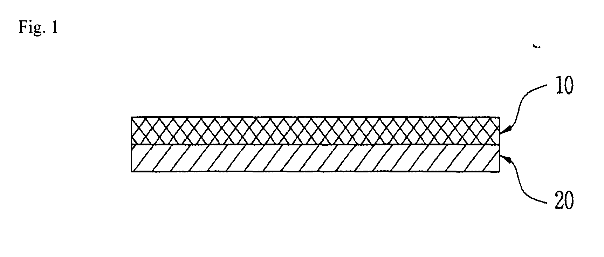

Fig. 1 is a sectional view of a sheet showing an application form of a hydrocolloid prepared according to the present invention.

## DETAILED DESCRIPTION OF THE INVENTION

The present invention is described in more detail below.

The present invention comprises at least a first step of preparing a pre-polymer, a second step of preparing a complex containing the pre-polymer, and a third step of coating on an object for coating process with the complex and polymerizing the complex in this state. Each step will be specifically explained as follows:

[First Step]

The first step comprises mixing an acryl monomer with an ultraviolet initiator and irradiating on the mixture with ultraviolet rays to prepare a pre-polymer having an appropriate viscosity The viscosity of pre-polymer is allowed to be in a range of 200 cps ~ 10,000 cps by regulating irradiation intensity and time of ultraviolet rays. If the viscosity is too low as much as less than 200 cps, the thickness is difficult to be regulated on coating. To obtain the desired thickness, coating must be repeated by several times, which is a troublesome matter. If the viscosity is too high as much as in excess of 10,000 cps, the mixing procedure in the second step is not easily occurred.

It is preferred to use monomers that Tg of the mixed system may be in a range of 0 °C ~ -50°C (below zero) by appropriately setting a mixing ratio of a monomer having a high glass transition temperature (Tg) and a monomer having a low Tg, as an acryl monomer. The term "glass transition temperature of the mixed system" is meant to a glass transition temperature that each monomer is polymerized to obtain a copolymer.

Examples of monomers having a high glass transition temperature include acrylic acid, methacrylic acid, methyl methacrylate, and the like, whereas examples of monomers having a low glass transition temperature include 2-ethylhexyl acrylate, butyl acrylate, isooctyl acrylate, hydroxybutyl acrylate, etha-caprolactone acrylate, and the like.

In addition, monomers having intermediate ranges (60 °C ~ -30°C (below zero)), for example, methylacrylate, hydroxyethyl methacylate, or hydroxypropyl acrylate, may be used as said acryl monomer.

Said ultraviolet initiator may be used by mixing one or two or more species selected from the group consisting of, for example, 1-hydroxycyclohexlphenyl ketone, 2,2-dimethoxy-2-phenyl-acetophenone, xanthone, benzaldehyde, anthraquinone, 3-methylacetophenone, 4-chlorobenzophenone, 4,4'-dimethoxy benzophenone, 4,4'-diaminobenzophenone, benzoin propyl ether, benzoin ethylether, 1-(4-isopropyl-phenol)-2-hydroxy-2-methyl propane-1-one, and thioxanthone. A commercially available initiator may be used by mixing one or two or more species selected from the group consisting of Irga-cure series from Ciba-Geigy (Swiss), Darocur series from Merck (Germany), and Lucirine series from BASF (Germany). Such ultraviolet initiator is not specifically limited, but preferably used by mixing 0.002 ~ 2 parts by weight relative to 100 parts by weight of an acryl monomer.

In said ultraviolet irradiation, an industrially available ultraviolet lamp such as a metal halide lamp, a mercury lamp, and a black light lamp may be usually used. Such ultraviolet irradiation may be performed in vacuo or in an inert gas such as argon and helium or air. Irradiation intensity and time of ultraviolet rays vary with species and amounts of the used monomers. If the ultraviolet rays are irradiated at an intensity of $0.01 \text{ mW/cm}^2$ ~ $5.0 \text{ mW/cm}^2$ for 1 ~ 180 seconds, a pre-polymer with a viscosity of 200 cps ~ 10,000 cps, preferably, 1,000 cps ~ 5,000 cps may be prepared.

In addition, the temperature of reactants (an acryl monomer + an ultraviolet initiator) is, preferably, kept at 30 °C or below. W hen the ultraviolet rays are irradiated, the reaction is initiated and the temperature is elevated by heat of reaction in the reactant. If the reaching temperature of reactant after irradiating ultraviolet rays raises 5 °C ~ 20°C, preferably, about 10°C more than the temperature prior to irradiation, the reaction is preferably terminated. The reason is because it is difficult to perform mixing of the second step and coating of the third step on the ground that if the temperature after irradiation raises much higher than the temperature prior to irradiation, that is, if the temperature after irradiation raises too high as much as in excess of 50°C (wherein the temperature before irradiation is 30°C), the product (pre-polymer) is changed into higher molecular weight material and has a high viscosity. Such termination of reaction may be attained by inhibiting a radical reaction using oxygen (referred to step 1-1).

[Second Step]

The second step comprises mixing the pre-polymer prepared in said first step with at least an ultraviolet photoinitiator and a high absorbent substance to prepare one complex. Said complex has, preferably, a viscosity of 500 cps ~ 50,000 cps.

The ultraviolet initiator to be used in the present second step may include those as illustrated in the first step. It is preferred that the initiator is mixed in an amount of 0.002 ~ 2 parts by weight, which is not specifically limited, relative to 100 parts by weight of pre-polymer.

Said high absorbent substance includes a liquid phase and a powder phase, and is preferably selected from those having an excellent absorbency for mucinoid exudates. It is preferred to use one or two or more mixture selected from the group consisting of, for example, polyacrylic acid salt, polymethacrylic acid salt, polyacrylamide, cellulose, carboxymethylcellulose, hydroxyethylcellulose, pectin, guar gum, sodium alginate, chitin, chitosane, gellatin, starch, xanthane gum and karaya gum. Such high absorbent substance is, preferably, mixed in a range of 0.2 ~ 100 parts by weight relative to 100 parts by weight of pre-polymer. If the high absorbent substance is contained too little as much as less than 0.2 parts by weight, it is difficult to exhibit the desired absorbency in the present invention. If it is contained too large as much as in excess of 100 parts by weight, the residue may be remained on the wound.

Said complex essentially contains a pre-polymer, an ultraviolet initiator and a high absorbent substance as described above, but may further comprise a cross linking agent for improving a shearing stress and cohesion. Said cross linking agent may be mixed in an amount of 0.0001 ~ 2 parts by weight relative to 100 parts by weight of pre-polymer.

In addition, said complex further comprises additives useful in treating the wound such as humectants, wound healing accelerators, antibacterial agents, and cell growth factors.

For said humectant and wound healing accelerator, hyaluronic acid, keratan, collagen, dermatan sulfate, heparin, heparin sulfate, sodium alginate, sodium carboxymethylcellulose, chondrotin sulfate, 3-aminopropyldihydrogen phosphate or oligopolysaccharide thereof, and the like may be employed alone or in a mixture thereof. For said antibacterial agent, gluconate chlorohexidin, acetate chlorohexidin, hydrochloride chlorohexidin, silver sulferdiazine. povidone iodine, benzalkonium chloride, furagin, idocaine, hexachlorophene, chlorotetracycline, neomycin, penicilline, gentamycin, acrinol, and silver (Ag) compounds, and the like may be used. In addition, for said cell growth factor, a plateletderived growth factor (PDGF), a transforming growth factor (TGF-β), a epidermal cell-derived growth factor (EGF), a fibroblast growth factor (FGF), and the like may be employed alone or in a mixture thereof. Each additive may be mixed in an amount of 0.001 ~ 0.5 parts by weight relative to 100 parts by weight of pre-polymer.

In addition, the complex may be transparent or semitransparent. Preferably, it is constructed to be transparent, for easy wound observation. In addition, the complex may include a pigment (preferably, skin color pigment). If possible, it may be used in a trace amount, among the amounts not interfering wound observation.

After mixing and preparing the complex with a composition above, eliminating the dissolved oxygen in said complex is preferably performed for the more efficient polymerization to be proceeded in the third step below (referred to Step 2-1). Specifically, the dissolved oxygen, which may be present in the complex, can inhibit the polymerization reaction to be proceeded in the third step. In case of using oxygen for completing the reaction of pre-polymer (for example, in case of practicing Step 1-1), a large quantity of the dissolved oxygen may be present in the complex. If possible, it is preferred to practice Step 2-1 in which the dissolved oxygen is eliminated, before the third step. The dissolved oxygen may be easily eliminated by inhaling an inert gas such as nitrogen, argon, and helium in the complex.

[Third Step]

The third step comprises coating the object with the complex, and then irradiating on the complex with ultraviolet rays to polymerize a pre-polymer. Specifically, the pre-polymer in the complex is polymerized in a state coated on the object to form a hydrocolloid type of acryl polymer. After coating the object for coating process with the complex, a clear film is covered on the coated complex, to make an inert gas atmosphere, followed by irradiating on the film with ultraviolet rays to be subjected to the polymerization.

As above, the pre-polymer is polymerized together with the high absorbent substance by the polymerization via ultraviolet irradiation in the third step, with forming interpenetrated polymer networks (IPNs). Thus, the high absorbent substance used for an excellent absorbency is not degraded, even if it absorbs exudates, and no residue is remained.

In addition, the ultraviolet irradiation intensity in the third step is, preferably, as low as in a range of 0.01 mW/cm$^2$ ~ 5.0 mW/cm$^2$. Such ultraviolet irradiation intensity improves adhesiveness, and moreover the increased molecular weight improves heat resistance. If the ultraviolet irradiation intensity is too low as much as less than 0.01 mW/cm$^2$, the pre-polymer and the high absorbent substance cannot form a good networks. If the ultraviolet irradiation intensity is too high as much as in excess of 5.0 mW/cm$^2$, a sticky property may be lowered and self-adhesiveness also dropped. The ultraviolet irradiation time is not specifically limited, but may be for 1 ~ 30 minutes at the irradiation intensity above.

The thus prepared hydrocolloid of the present invention is usefully applied to skin, particularly, wounds. Specifically, the hydrocolloid may be usefully used in a fixing adhesive tape for fixing medical apparatuses or bandages on skin, and preferably, wound dressings.

Fig. 1 illustrates a sheet, which may be usefully used as wound dressing, showing an application form of a hydrocolloid prepared according to the present invention. The sheet comprises at least self-adhesive absorbent layer (10). The absorbent layer (10) consists of an acryl polymer (hydrocolloid) polymerized after coating on the object in the third step. The absorbent layer (10) is a transparent layer or a semitransparent layer, preferably, a transparent layer.

As shown in Fig. 1, the sheet has, preferably, a substrate (20) bonded to any one side of the absorbent layer (10). It is preferred that the substrate (20) is transparent and flexible. The substrate (20) may be web, non-woven and resin films. Preferably, it is a resin film having liquid impermeability (waterproof) as well as gas permeability (moisture permeability). The resin film that may be preferably used as a substrate (20) is a resin being able to give high moisture permeability, and examples of this resin include a film prepared using polyurethane, polyethylene, silicon resins, natural and synthetic rubbers, polyglycolic acid, polylactic acid or copolymers thereof, synthetic polymers such as polyvinylalcohol, and polyvinylpyrrolidone, natural polymer such as collagen, gelatin, hyaluronic acid, soduim alginate, chitin, chitosan, fibrin, and cellulose, or synthetic polymer derived from these alone or in mixtures thereof. More preferably, it is a polyurethane film. The polyurethane film is so desirable that it may have an excellent physical property such as tensile strength, and tensile force as well as gas permeability (moisture permeability) and liquid impermeability (waterproof). The polyurethane film includes one prepared by extrusion, solvent volatilization or coagulation.

The size or form of such sheet is not restricted. For example, the sheet may be provided as cut from the prepared sheet above in a certain size for convenient use, or may be provided in a form of roll that users can directly cut it in a size fitted for a certain use. In addition, the sheet may have a structure that a release paper is bonded thereto. The release paper is positioned on the opposite side to the bonding side of substrate (20) and removed when the sheet is adhered to the skin (wound).

As used herein, the term "an object for coating process" includes those provided with surfaces that the complex can be coated. The object for coating process may be selected from the group consisting of the substrate (20) or the release paper, and a glass substrate, a metal substrate, a plastic substrate, and the like. Preferably, the object has a releasing property.

Specific experimental example and comparative examples are explained below. These examples are provided to illustrate the present invention in detail, and are not intended to restrict the technical scope of the present invention.

## EXAMPLES

< First step: Preparation Example of Pre-polymer>

Under nitrogen atmosphere, 95 g of 2-ethylhexyl acrylate as an acryl monomer, 5 g of acrylic acid, and 0.2 g of Irgacure-184 (available from Ciba-Geigy AG, Swiss) as an ultraviolet initiator were added to a glass reactor, and the mixture was sufficiently stirred. Ultraviolet rays were irradiated on the reactant at an irradiation intensity of 1.0 mW/cm$^2$ for 30 seconds, using an ultraviolet lamp (Black light lamp) and then subjected to the reaction. When the temperature of the reactant was raised to 10 °C, the reaction was terminated by blowing oxygen in the reactor to obtain a pre-polymer. The obtained pre-polymer had a viscosity of 2,000 cps at 25 °C

< Second step: Preparation Example of Complex >

Under nitrogen atmosphere, 0.4 g of Irgacure-184 (available from Ciba-Geigy AG, Swiss) as an ultraviolet initiator, and 7 g of carboxymethylcellulose (KDA 6M15, available from Koje Co., Korea) and 15 g of polyacrylic acid salt (HS-1500, available from Songwon Industrial Co, Ltd., Korea) as high absorbent substances were added, in addition to 100 g of the pre-polymer obtained in Preparation Example of First step above, to a mixer and the mixture was stirred and dispersed for 30 minutes to obtain a viscous complex. The obtained complex had a viscosity of 20,000 cps at 25 °C.

< Third step: Examples 1~12 >

The complex prepared in Preparation Example of Second step above was coated in a thickness of 500 $\mu$m on a silicon-coated release paper with a knife-coater apparatus, followed by covering with PET film thereon. Ultraviolet rays were irradiated on each reactant at an irradiation intensity set forth in Table 1 below for 20 minutes, using an ultraviolet lamp (Black light lamp) and then sufficiently subjected to the reaction. After each covered PET film was peeled off, each 25 $\mu$m polyurethane film was laminated thereon to prepare self adhesive sheet specimens.
Physical properties of the specimens prepared above were measured by the methods illustrated below, and the results were represented in Table 1 below.

① 180° peeling adhesive strength measurement

Measurement of adhesive strength was tested according to JIS Z 0237, using a tensile test machine (Universal Test Machine, Tinus Olsen Korea). Specifically, the longitudinal edge of stainless steel test plate was met with that of a specimen set the adhesive side downward to the test plate. Then, the test plate was positioned on the longitudinal center of specimen and a paper was adhered to the adhesive side of about 125 mm part in the remaining specimen. Next, the specimen was compressed by running once a roller on the specimen at a speed of about 300 mm/min. After 20 minutes or so, the completely compressed specimen was peeled by about 25 mm, and peeled at a tensile speed of 300 mm/min. Each average load value between the time of peeling by 20 mm from the start of peeling and that of peeling by 80 mm was calculated by an integrator, and the value was used as a measurement of adhesive strength.

② Measurement of Absorbency

The prepared specimen was cut in a size of 2 by 2 cm and its initial weight (W1) was measured. The specimen was added to distilled water, its weight (W2) was measured after 48 h. The degree of absorbency was calculated by the following formula:

$$\text{Absorbency (\%)} = (W2 - W1)/W1 \times 100$$

③ Measurement of Retention

The term "retention" means the ability that the specimen is not degraded by a physiological saline. The measurement of retention is performed by measuring the weight percentage of specimen maintained after exposing to the physiological saline under the conditions set forth below. 100% of retention means that when the specimen is adhered to a wound and then exchanged, no residue is remained on the wound site.
First, the specimen was cut in a diameter of 2.54 cm, and its initial weight (W1) was measured and recorded. Next, the specimen was soaked in a physiological saline and stirred for 18 h. The specimen was taken out, and dried in a thermohygrostat of temperature 50°C×50% RH for 72 h. The weight (Wf) of specimen was measured. The degree of retention (%) was calculated by the following formula:

$$\text{Retention (\%)} = (Wf/Wi) \times 100$$

[Comparative Example 1]

A Comfeel product from Coloplast Corp. (US) was used as a specimen in this comparative example. This product was prepared by a method described in US Patent No. 4,231,369. The physical properties of the specimen were evaluated by the same method as the Examples above, and the results were represented in Table 1 below.

[Comparative Example 2]

A Duoderm product from Convatec (US) was used as a specimen in this comparative example. This product was prepared by a method described in US Patent No. 4,551,490. The physical properties of the specimen were evaluated by the same method as the Examples above, and the results were represented in Table 1 below.

Table 1: Results of evaluating Physical Properties

| Class | Ultraviolet Irradiation Intensity (mW/cm$^2$) | Adhesive Strength (kgf/25 mm) | Absorbency (%) | Retention (%) |
|---|---|---|---|---|
| Example 1 | 0.01 | 3.5 | 690 | 100 |
| Example 2 | 0.05 | 3.5 | 700 | 100 |
| Example 3 | 0.1 | 3.4 | 690 | 100 |
| Example 4 | 0.5 | 2.9 | 710 | 100 |
| Example 5 | 1 | 2.1 | 700 | 100 |
| Example 6 | 1.5 | 2.0 | 700 | 100 |
| Example 7 | 2 | 1.8 | 710 | 100 |
| Example 8 | 2.5 | 2.7 | 700 | 100 |
| Example 9 | 3 | 3.1 | 710 | 100 |
| Example 10 | 5 | 3.1 | 700 | 100 |
| Example 11 | 7 | 1.9 | 690 | 100 |
| Example 12 | 10 | 1.6 | 700 | 100 |
| Comp.Example 1 | - | 1.3 | 410 | 78 |
| Comp.Example 2 | - | 1.2 | 220 | 91 |

As can be seen from the Table 1, the sheets (Examples 1~12) applied by the hydrocolloids according to the present invention has a very excellent absorbency and an excellent adhesive strength, without adding low molecular weight material as a tackifier, compared to existing products on the market (Comparative Example 1, 2). It can be seen from the results of Examples that the adhesive strength is easily regulated by the irradiation intensity of ultraviolet rays.
In addition, the retentions of Comparative Examples 1 and 2 are 78% and 91%, respectively, but all the retentions of Examples in the present invention are 100%. Such excellent retention is because the interpenetrated polymer networks (IPNs) are formed from the aryl polymers and the high absorbent substances. This fact means that even though the sheet absorbs exudates, it is not degraded, so that no residue is remained.

**INDUSTRIAL APPLICABILITY**

The present invention has an effect that a hydrocolloid may be easily prepared without using a large quantity of solvent or other additives. In addition, the present invention has effects that a hydrocolloid prepared according to the present invention has an excellent absorbency as well as self-adhesiveness, is not allowed to leave residues upon being removed from the skin (wound) and has low skin stimuli due to no use of low molecular weight tackifier.

**Claims**

1. A method for a hydrocolloid which comprises
   a first step of preparing a pre-polymer having a viscosity of 200 cps ~ 10,000 cps by mixing an acryl monomer and an ultraviolet initiator and irradiating on the mixture with ultraviolet rays;
   a second step of preparing a complex by mixing the resulting pre-polymer with at least an ultraviolet initiator and a high absorbent substance; and

a third step of polymerizing the resulting complex by coating on an object for coating process with the complex and irradiating the coated complex with ultraviolet rays.

2. The method for preparing a hydrocolloid according to claim 1, which further comprises a step 1-1 of inhibiting the polymerization using oxygen, when the temperature after ultraviolet irradiation raises 5 °C ~ 20 °C more than the temperature before irradiation in the first step of preparing a pre-polymer.

3. The method for preparing a hydrocolloid according to claim 2, which further comprises a step 2-1 of eliminating oxygen in the complex prepared in the second step using an inert gas, before the third step.

4. The method for preparing a hydrocolloid according to claim 1, wherein the ultraviolet irradiation intensity in the third step is in a range of 0.01 mW/cm$^2$ ~ 5.0 mW/cm$^2$.

5. The method for preparing a hydrocolloid according to claim 1, wherein the high absorbent substance in the second step comprises one or two or more mixtures selected from the group consising of polyacrylic acid salt, polymethacrylic acid salt, polyacrylamide, cellulose, carboxymethylcellulose, hydroxyethylcellulose, pectin, gua gum, sodium alginate, chitin, chitosan, gellatin, starch, xantan gum and karaya gum.

Fig. 1

10

20

**European Patent Office**

# EUROPEAN SEARCH REPORT

Application Number

EP 07 01 0166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 536 778 A (KRECKEL KARL W [DE] ET AL) 16 July 1996 (1996-07-16) <br> * column 12, line 20 - line 35; example 1 * <br> * column 6, line 25 - line 37 * <br> * column 6, line 43 - line 47 * <br> * column 3, line 50 - line 60 * | 1-5 | INV. <br> C08F251/00 <br> C08F251/02 <br> C08F265/04 <br> C08F265/06 <br> C08F283/00 <br> C08L51/02 <br> C09D151/02 <br> C09J151/02 <br> C08L1/00 <br> C08L3/00 <br> C08L5/00 |
| X | EP 0 736 585 A1 (MINNESOTA MINING & MFG [US]) 9 October 1996 (1996-10-09) <br> * page 5 - page 6; example 1 * <br> Comparative example 3 | 1-5 | |
| A | WO 02/094328 A2 (BASF AG [DE]; WHITMORE DARRYL L [US]; ENGELHARDT FRIEDRICH [DE]) 28 November 2002 (2002-11-28) <br> * page 21 - page 29 * <br> * page 32, line 18 - line 33 * <br> * page 37 - page 38; example 1 * | 1,3,5 | |
| A | US 2002/026005 A1 (MUNRO HUGH SEMPLE [GB]) 28 February 2002 (2002-02-28) <br> * paragraphs [0071], [0116], [0117], [0134] * | 1,4,5 | TECHNICAL FIELDS SEARCHED (IPC) <br><br> C08F <br> C08L <br> C09D |
| A | US 2005/070688 A1 (LEWANDOWSKI KEVIN M [US] ET AL) 31 March 2005 (2005-03-31) <br> * paragraphs [0053], [0115], [0123] * | 2,5 | C09J <br> A61L <br> A61K |
| A | US 6 689 379 B1 (BRACHT STEFAN [DE]) 10 February 2004 (2004-02-10) <br> * column 6, line 53 - column 7, line 7; claims 1,20 * | 5 | |

The present search report has been drawn up for all claims

| Place of search | Date of completion of the search | Examiner |
|---|---|---|
| The Hague | 9 August 2007 | Dalet, Pierre |

## ANNEX TO THE EUROPEAN SEARCH REPORT
## ON EUROPEAN PATENT APPLICATION NO.

EP 07 01 0166

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2007

| Patent document cited in search report | | Publication date | Patent family member(s) | | Publication date |
|---|---|---|---|---|---|
| US 5536778 | A | 16-07-1996 | AU | 678969 B2 | 19-06-1997 |
| | | | AU | 4111793 A | 29-11-1993 |
| | | | CA | 2133653 A1 | 11-11-1993 |
| | | | DE | 4214507 A1 | 04-11-1993 |
| | | | EP | 0638096 A1 | 15-02-1995 |
| | | | JP | 7506387 T | 13-07-1995 |
| | | | WO | 9322354 A1 | 11-11-1993 |
| EP 0736585 | A1 | 09-10-1996 | AU | 5320896 A | 23-10-1996 |
| | | | BR | 9604804 A | 09-06-1998 |
| | | | CA | 2214630 A1 | 10-10-1996 |
| | | | CN | 1179788 A | 22-04-1998 |
| | | | DE | 69511843 D1 | 07-10-1999 |
| | | | DE | 69511843 T2 | 29-06-2000 |
| | | | JP | 11503191 T | 23-03-1999 |
| | | | WO | 9631564 A1 | 10-10-1996 |
| WO 02094328 | A2 | 28-11-2002 | NONE | | |
| US 2002026005 | A1 | 28-02-2002 | AU | 762658 B2 | 03-07-2003 |
| | | | AU | 2306100 A | 25-08-2000 |
| | | | CA | 2361871 A1 | 10-08-2000 |
| | | | EP | 1150721 A1 | 07-11-2001 |
| | | | WO | 0045864 A1 | 10-08-2000 |
| | | | JP | 2002536073 T | 29-10-2002 |
| US 2005070688 | A1 | 31-03-2005 | AT | 358687 T | 15-04-2007 |
| | | | EP | 1668052 A1 | 14-06-2006 |
| | | | JP | 2007506838 T | 22-03-2007 |
| | | | US | 2006292209 A1 | 28-12-2006 |
| | | | WO | 2005035607 A1 | 21-04-2005 |
| US 6689379 | B1 | 10-02-2004 | AT | 364379 T | 15-07-2007 |
| | | | AU | 779960 B2 | 24-02-2005 |
| | | | AU | 4294200 A | 10-11-2000 |
| | | | BR | 0011131 A | 12-08-2003 |
| | | | CA | 2370019 A1 | 02-11-2000 |
| | | | CN | 1354654 A | 19-06-2002 |
| | | | CZ | 20013763 A3 | 13-02-2002 |
| | | | DE | 19918106 A1 | 26-10-2000 |
| | | | WO | 0064418 A2 | 02-11-2000 |
| | | | EP | 1171104 A2 | 16-01-2002 |
| | | | HK | 1045264 A1 | 16-09-2005 |
| | | | HU | 0200635 A2 | 29-07-2002 |
| | | | JP | 2002542277 T | 10-12-2002 |
| | | | MX | PA01010504 A | 04-06-2002 |

For more details about this annex : see Official Journal of the European Patent Office, No. 12/82

EPO FORM P0459

**ANNEX TO THE EUROPEAN SEARCH REPORT
ON EUROPEAN PATENT APPLICATION NO.**

EP 07 01 0166

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2007

| Patent document cited in search report | Publication date | Patent family member(s) | Publication date |
|---|---|---|---|
| US 6689379 B1 | | NZ 514946 A | 27-02-2004 |
| | | PL 352550 A1 | 25-08-2003 |
| | | RU 2242971 C2 | 27-12-2004 |
| | | TR 200102916 T2 | 21-01-2002 |
| | | ZA 200108564 A | 11-09-2002 |

EPO FORM P0459

For more details about this annex : see Official Journal of the European Patent Office, No. 12/82

## REFERENCES CITED IN THE DESCRIPTION

**Patent documents cited in the description**

- US 3339546 A
- US 4231369 A
- US 4551490 A
- US 6583220 B
- US 4477325 A
- US 4738257 A
- US 6903151 B